# EUROPEAN PATENT APPLICATION

(11) **EP 4 825 961 A1**
(43) Date of publication of application: **21.10.2026**
(21) Application number: 24903752.4
(22) Date of filing: 12.12.2024
(51) Int. Cl.: C12N 5/10, C12N 1/00, C12N 15/12

(54) **METHOD FOR INDUCING HEPATIC STEM CELLS OR HEPATIC PROGENITOR CELLS**

(30) Priority: 12.12.2023 JP 2023209437
(71) Applicant: Kyushu University, National University Corporation, Nishi-ku Fukuoka-shi Fukuoka 819-0395 (JP)
(72) Inventor: SUZUKI Atsushi, Fukuoka-shi, Fukuoka 819-0395 (JP)
(74) Representative: J A Kemp LLP
(86) International application number: PCT/JP2024/043976
(87) International publication number: WO 2025/127099

(57) **Abstract**

The present invention provides, for example, a method for inducing a hepatic stem cell or a hepatic progenitor cell from a non-hepatic stem cell or a non-hepatic progenitor cell, a method for producing a hepatic stem cell or a hepatic progenitor cell, and a method for producing a hepatocyte or a cholangiocyte.

## Description

### Technical Field

The present invention relates to, for example, a method for inducing a hepatic stem cell or a hepatic progenitor cell from a non-hepatic stem cell or a non-hepatic progenitor cell, a method for producing a hepatic stem cell or a hepatic progenitor cell, and a method for producing a hepatocyte or a cholangiocyte.

### Background Art

Techniques for preparing hepatocytes by introducing a certain gene set as reprogramming factors to human fibroblasts have already been known. For example, a method of preparing induced hepatocytes from non-hepatic cells using reprogramming factors such as CEBPA, HNF4A, FOXA3, GATA4, and HNF1A is known (Patent Literature 1).

Moreover, the reprogramming of mouse fibroblasts into hepatic stem cells by the introduction of Hnf1β and Foxa3 (Non Patent Literature 1), a method for reprogramming human fibroblasts into mature hepatocytes by causing the expression of FOXA3, HNF1A, and HNF4A (Non Patent Literature 2), a method for preparing human induced hepatocytes by causing the overexpression of HNF1A, HNF4A, and HNF6 together with maturation factors ATF5, PROX1, and CEBPA (Non Patent Literature 3), and the like are known.

However, the hepatocytes induced in Patent Literature 1 and Non Patent Literatures 2 and 3 have poor ability to proliferate and cannot differentiate into cholangiocytes. Furthermore, Non Patent Literature 1 provides data on mouse cells and does not involve human cells.

### Citation List

### Patent Literature

Patent Literature 1: JP 2015-527084A

### Non Patent Literature

Non Patent Literature 1: Bing Yu et al., Cell Stem Cell, 13 (3): 328-40, 2013
Non Patent Literature 2: Pengyu Huang et al., Cell Stem Cell, 14 (3): 370-384, 2014
Non Patent Literature 3: Yuanyuan Du et al., Cell Stem Cell, 14 (3), 394-403, 2014

### Summary of Invention

In one embodiment, an object of the present invention is to provide a method for inducing a hepatic stem cell or a hepatic progenitor cell or a method for producing a hepatic stem cell or a hepatic progenitor cell.

The present inventor has found that a hepatic stem cell or a hepatic progenitor cell can be induced from a non-hepatic stem cell or a non-hepatic progenitor cell by using a combination of specific reprogramming factors. The present invention encompasses the following embodiments.

(1) A method for inducing a hepatic stem cell or a hepatic progenitor cell from a non-hepatic stem cell or a non-hepatic progenitor cell, comprising
   introducing a combination comprising:
   HNF1 gene or HNF1 protein, FOXA gene or FOXA protein, and a Wnt signal activator to the non-hepatic stem cell or the non-hepatic progenitor cell.
(2) A method for producing a hepatic stem cell or a hepatic progenitor cell, comprising
   inducing the hepatic stem cell or the hepatic progenitor cell from a non-hepatic stem cell or a non-hepatic progenitor cell by introducing a combination comprising:
   HNF1 gene or HNF1 protein, FOXA gene or FOXA protein, and a Wnt signal activator to the non-hepatic stem cell or the non-hepatic progenitor cell.
(3) The method according to (1) or (2), wherein the combination consists of the HNF1 gene or the HNF1 protein, the FOXA gene or the FOXA protein, and the Wnt signal activator.
(4) The method according to any of (1) to (3), wherein HNF1 is HNF1A.
(5) The method according to any of (1) to (4), wherein FOXA is FOXA3.
(6) The method according to any of (1) to (5), wherein the Wnt signal activator is CHIR99021 or BIO (6-bromoindirubin-3-oxime).
(7) The method according to (6), wherein the Wnt signal activator is CHIR99021.
(8) The method according to any of (1) to (7), wherein the non-hepatic stem cell or the non-hepatic progenitor cell is a vascular endothelial cell or a blood-derived cell.
(9) The method according to (8), wherein the vascular endothelial cell is derived from umbilical vein, peripheral blood, or umbilical blood.
(10) The method according to (8), wherein the blood-derived cell is a peripheral blood T cell or an umbilical blood T cell.
(11) The method according to any of (1) to (10), wherein the combination further comprises HNF6 gene or HNF6 protein.
(12) A method for producing a hepatocyte or a cholangiocyte, comprising the steps of:
   (a) inducing a hepatic stem cell or a hepatic progenitor cell by a method according to any of (1) and (3) to (11); and
   (b) allowing the induced hepatic stem cell or hepatic progenitor cell to differentiate into the hepatocyte or the cholangiocyte.
(13) A combination, or a kit or a composition comprising a combination, the combination comprising:
   HNF1 gene or HNF1 protein, FOXA gene or FOXA protein, and a Wnt signal activator.
(14) The combination, or the kit or the composition comprising the combination according to (13), wherein the combination further comprises HNF6 gene or HNF6 protein.
(15) The combination, or the kit or the composition comprising the combination according to (13) or (14) for use in a method according to any of (1) to (11).

### Advantageous Effects of Invention

The present invention provides, for example, a method for inducing a hepatic stem cell or a hepatic progenitor cell from a non-hepatic stem cell or a non-hepatic progenitor cell, a method for producing a hepatic stem cell or a hepatic progenitor cell, and a method for producing a hepatocyte or a cholangiocyte.

### Brief Description of Drawings

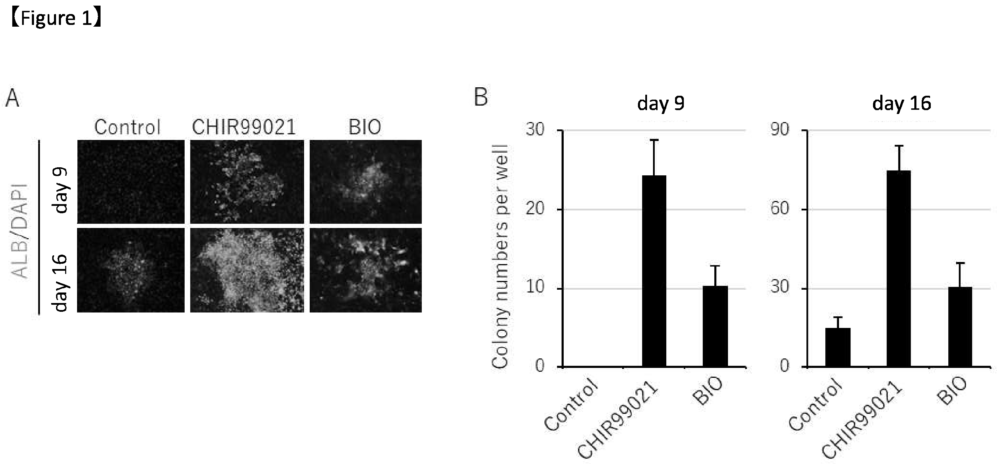

[Figure 1] Figure 1 shows results of introducing human iHepPC inducing factors (FOXA3, HNF1A, and HNF6) to HUVEC, then adding a Wnt activator, CHIR99021 or BIO to a culture solution, and performing albumin (ALB) immunostaining (A) and colony number counting (B) of formed colonies on days 9 and 16. DNA of the cells was stained with DAPI (blue).

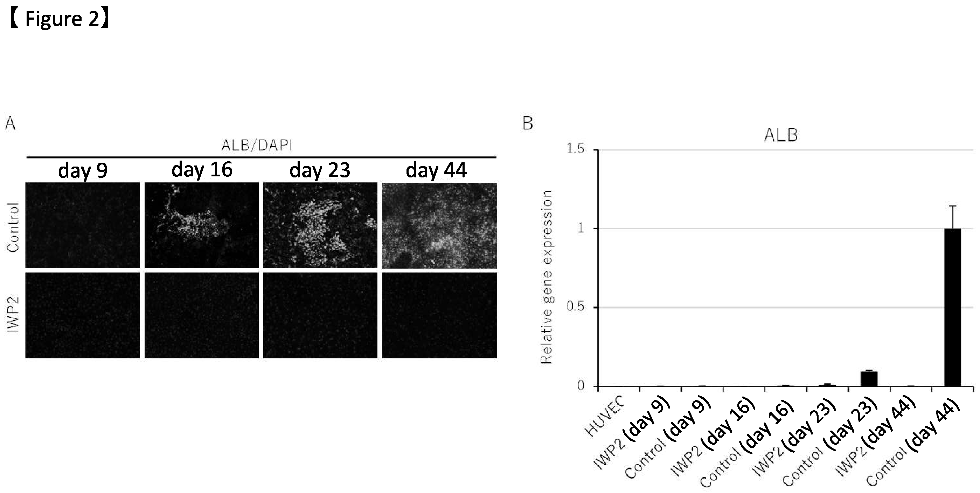

[Figure 2] Figure 2 shows results of introducing human iHepPC inducing factors (FOXA3, HNF1A, and HNF6) to HUVEC, then adding a Wnt inhibitor, IWP2 to a culture solution, and performing albumin (ALB) immunostaining (A) and ALB gene expression analysis (B) of formed colonies on days 9, 16, 23, and 44. A group supplemented with no Wnt signal inhibitor was expressed as Control. DNA of the cells was stained with DAPI (blue).

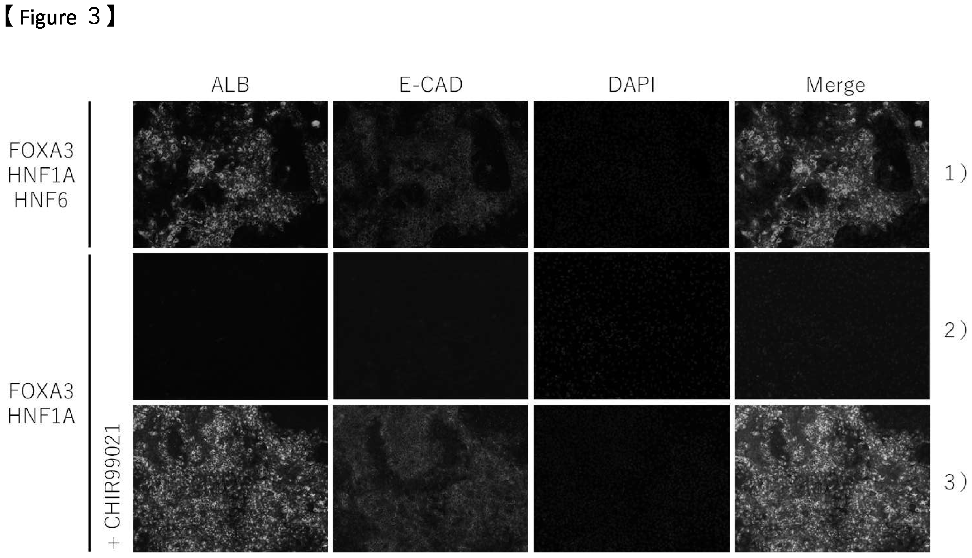

[Figure 3] Figure 3 shows results of performing expression analysis on albumin (ALB) and E-cadherin (E-CAD) in three groups: a group 1) in which human iHepPC inducing factors (FOXA3, HNF1A, and HNF6) were introduced to HUVEC, a group 2) in which some of the human iHepPC inducing factors (FOXA3 and HNF1A) were introduced thereto, and a group 3) in which some of the human iHepPC inducing factors (FOXA3 and HNF1A) were introduced thereto, followed by the addition of a Wnt signal activator (CHIR99021: 3 µM) to a culture solution. DNA of the cells was stained with DAPI (blue).

### Description of Embodiments

In one embodiment, the present invention relates to a method for inducing a hepatic stem cell or a hepatic progenitor cell from a non-hepatic stem cell or a non-hepatic progenitor cell, comprising introducing a combination comprising: HNF1 gene or HNF1 protein, FOXA gene or FOXA protein, and a Wnt signal activator to the non-hepatic stem cell or the non-hepatic progenitor cell.

In one embodiment, the present invention relates to a method for producing a hepatic stem cell or a hepatic progenitor cell, comprising inducing the hepatic stem cell or the hepatic progenitor cell from a non-hepatic stem cell or a non-hepatic progenitor cell by introducing a combination comprising: HNF1 gene or HNF1 protein, FOXA gene or FOXA protein, and a Wnt signal activator to the non-hepatic stem cell or the non-hepatic progenitor cell.

As used herein, the "non-hepatic stem cell" and the "non-hepatic progenitor cell" mean a cell other than the "hepatic stem cell" and the "hepatic progenitor cell" to be induced or produced by the method as described herein. Examples of the "non-hepatic stem cell" and the "non-hepatic progenitor cell" include fibroblasts, endothelial cells, blood cells, umbilical blood cells, bone marrow cells, keratinocytes, hepatocytes, cholangiocytes, myofibroblasts, nervous system cells, and epithelial cells. For example, a vascular endothelial cell or a blood-derived cell may be used as the "non-hepatic stem cell" and the "non-hepatic progenitor cell". Examples of the vascular endothelial cell include those derived from umbilical vein, peripheral blood, or umbilical blood. Examples of the blood-derived cell include peripheral blood cells and umbilical blood cells (e.g., peripheral blood T cells and umbilical blood T cells). Examples of the "non-hepatic stem cell" and the "non-hepatic progenitor cell" as used herein include cells derived from mammals such as mice, rats, rabbits, cats, dogs, monkeys, and humans. In one embodiment, the "non-hepatic stem cell" and the "non-hepatic progenitor cell" are human-derived cells.

As used herein, not only embryonic cells but adult-derived cells may be used as the "non-hepatic stem cell" and the "non-hepatic progenitor cell". Thus, the method as described herein also achieves liver function tests or drug screening using adult-derived cells, for example, peripheral blood-derived hepatic stem cells or hepatic progenitor cells.

As used herein, the "hepatic stem cell" or the "hepatic progenitor cell" means a cell having the ability to differentiate into both of a hepatocyte and a cholangiocyte, and self-propagating capacity or self-renewal capacity. Examples of markers of the hepatocyte and the hepatic progenitor cell include albumin and E-cadherin.

The combination as described herein may be a combination of reprogramming factors. As used herein, the "reprogramming" means a process of changing the differentiated state of a cell to a differentiated state different from that of the cell or an undifferentiated state. As described herein, the reprogramming may mean that the "hepatic stem cell" and the "hepatic progenitor cell" are induced from the "non-hepatic stem cell" and the "non-hepatic progenitor cell". In the method as described herein, the "hepatic stem cell" and the "hepatic progenitor cell" may be induced from the "non-hepatic stem cell" and the "non-hepatic progenitor cell" without involving a pluripotent stem cell. Examples of factors for use in such reprogramming (reprogramming factors) include a combination comprising or consisting of HNF1 gene or HNF1 protein, FOXA gene or FOXA protein, and a Wnt signal activator. Specific examples of the combination include any of following combinations 1) to 4), for example, a combination 1) or 2):
1) a combination of the HNF1 gene, the FOXA gene, and the Wnt signal activator,
2) a combination of the HNF1 protein, the FOXA protein, and the Wnt signal activator,
3) a combination of the HNF1 gene, the FOXA protein, and the Wnt signal activator, and
4) a combination of the HNF1 protein, the FOXA gene, and the Wnt signal activator.

HNF1 (hepatocyte nuclear factor 1) is a protein having a homeodomain and has two types of isoforms, HNF1A and HNF1B.

FOXA is a hepatocyte nuclear factor (transcriptional factor) necessary for the earliest stage of liver tissue formation and includes FOXA1, FOXA2, and FOXA3. These FOXA transcriptional factors are considered to have functional complementarity to each other because of having 90% or higher homology at the amino acid level in a common forkhead/winged helix domain.

The combination as described herein may comprise an additional factor without inhibiting an effect of the method as described herein, for example, the induction of a hepatic stem cell or a hepatic progenitor cell or the production of a hepatic stem cell or a hepatic progenitor cell. Examples of such a factor include MYC. MYC family genes are known as transcriptional factors that work by binding to nuclear DNA, and include c-MYC, L-MYC, and N-MYC in humans.

Table 1 shows the amino acid sequences of the reprogramming factors and the nucleotide sequences of genes encoding these factors.

**[Table 1]**

| Reprogramming factor | Nucleotide sequence | Amino acid sequence |
|---|---|---|
| HNF1A NM_000545 | SEQ ID NO: 1 | SEQ ID NO: 2 |
| FOXA1 NM_004496 | SEQ ID NO: 3 | SEQ ID NO: 4 |
| FOXA2 NM_021784 | SEQ ID NO: 5 | SEQ ID NO: 6 |
| FOXA3 NM_004497 | SEQ ID NO: 7 | SEQ ID NO: 8 |

The genes encoding these factors or their fragments may be cloned with reference to, for example, "Molecular Cloning, A Laboratory Manual (4th edition)" (Cold Spring Harbor Laboratory Press (2012)) or may be obtained from addgene and the like.

As described herein, the gene encoding HNF1A is referred to as "HNF1A gene", and the gene encoding HNF6 is referred to as "HNF6 gene". Genes encoding other factors may also be expressed as described above.

The reprogramming factors (genes or proteins) as used herein are not limited by nucleic acid sequences having the nucleotide sequences represented by SEQ ID NOs shown in Table 1 or amino acid sequences having the amino acid sequences represented thereby. The following mutant forms may also be used as long as the mutant forms have functions of the reprogramming factors in the method as described herein:
(a) a protein that consists of an amino acid sequence comprising the amino acid sequence shown in Table 1 (amino acid sequence represented by SEQ ID NO: 2, 4, 6, or 8) having deletion, substitution, or addition of one or several (e.g., 10 or less, 5 or less, 4 or less, 3 or less, or 2) amino acids, and has functions as a reprogramming factor;
(b) a protein that consists of an amino acid sequence having 80% or higher, 85% or higher, 90% or higher, 95% or higher, 98% or higher, or 99% or higher (e.g., 90% or higher) sequence identity to the amino acid sequence shown in Table 1 (amino acid sequence represented by SEQ ID NO: 2, 4, 6, or 8), and has functions as a reprogramming factor;
(c) a nucleic acid encoding the protein (a);
(d) a nucleic acid encoding the protein (b); and
(e) a nucleic acid that hybridizes under stringent conditions with a nucleic acid consisting of a nucleotide sequence complementary to the nucleotide sequence shown in Table 1 (amino acid sequence represented by SEQ ID NO: 1, 3, 5, or 7), and encodes a protein having functions as a reprogramming factor.

As used herein, the "stringent conditions" may be, for example, conditions of 5 × SSC, 5 × Denhardt's solution, 0.5% SDS, 50% formamide, and 50°C. Under these conditions, it can be expected that DNA and/or RNA having high homology can be obtained more efficiently with increase in temperature. However, possible elements that influence the stringency of hybridization are plurality of elements such as temperatures, DNA and/or RNA concentrations, DNA and/or RNA length, ionic strength, times, and salt concentrations. Those skilled in the art are capable of achieving similar stringency by appropriately selecting these elements.

Whether or not a specific protein has functions as a reprogramming factor can be tested, for example, as described in Examples of the present specification. For example, the combination as described herein comprising the specific protein is introduced to non-hepatic stem cells or non-hepatic progenitor cells, which are then cultured for a predetermined period. The specific protein can be examined on the basis of whether or not the expression of hepatic stem cell or hepatic progenitor cell markers, for example, albumin, α-fetoprotein, and E-cadherin, is increased in the cells.

The type of the Wnt signal activator as described herein is not limited. Examples thereof include proteins belonging to the Wnt family (e.g., Wnt1), Wnt receptors, Wnt receptor agonists, and glycogen synthase kinase-3 (GSK-3) inhibitors, for example, CHIR99021 (CAS No. 252917-06-9), BIO (CAS No. 6-bromoindirubin-3-oxime) (667463-62-9), IQ-1 (CAS No. 331001-62-8), and BML284 (CAS No. 853220-52-7). The Wnt signal activator may be, for example, CHIR99021 or BIO. The concentration of the Wnt signal activator is not limited and may be, for example, 0.1 µM or higher, 0.2 µM or higher, 0.5 µM or higher, or 1 µM or higher and can be 50 µM or lower, 30 µM or lower, 10 µM or lower, or 5 µM or lower. The concentration of the Wnt signal activator may be, for example, approximately 1 µM or 3 µM.

The combination as described herein may be, for example, but not limited to, any of following combinations (1) to (5), for example, a combination (2) or (3):
(1) a combination of (a) the HNF1A gene or the HNF1A protein, (b) the FOXA3 gene or the FOXA3 protein, and (c) CHIR99021 or BIO,
(2) a combination of (a) the HNF1A gene, (b) the FOXA3 gene, and (c) CHIR99021,
(3) a combination of (a) the HNF1A protein, (b) the FOXA3 protein, and (c) CHIR99021,
(4) a combination of (a) the HNF1A gene, (b) the FOXA3 gene, and (c) BIO, and
(5) a combination of (a) the HNF1A protein, (b) the FOXA3 protein, and (c) BIO.

The combination may comprise these factors or may consist of or consist essentially of these factors.

The combination as described herein may further comprise an additional factor, for example, HNF6 gene or HNF6 protein.

The combination as described herein may be, for example, but not limited to, any of following combinations (1) to (5), for example, a combination (2) or (3):
(1) a combination of (a) the HNF1A gene or the HNF1A protein, (b) the FOXA3 gene or the FOXA3 protein, (c) CHIR99021 or BIO, and (d) the HNF6 gene or the HNF6 protein,
(2) a combination of (a) the HNF1A gene, (b) the FOXA3 gene, (c) CHIR99021, and (d) the HNF6 gene,
(3) a combination of (a) the HNF1A protein, (b) the FOXA3 protein, (c) CHIR99021, and (d) the HNF6 protein,
(4) a combination of (a) the HNF1A gene, (b) the FOXA3 gene, (c) BIO, and (d) the HNF6 gene, and
(5) a combination of (a) the HNF1A protein, (b) the FOXA3 protein, (c) BIO, and (d) the HNF6 protein.

The combination may comprise these factors or may consist of or consist essentially of these factors.

In general, low-molecular compounds are easier to handle than genes or proteins. Thus, when the Wnt signal activator is a low-molecular compound, reprogramming may be more conveniently performed by replacing the gene or the protein for use as a reprogramming factor with the Wnt signal activator.

Use of the Wnt signal activator may improve the induction efficiency of hepatic stem cells or hepatic progenitor cells in the method for inducing a hepatic stem cell or a hepatic progenitor cell or the method for producing a hepatic stem cell or a hepatic progenitor cell. Thus, the method as described herein may have high induction efficiency of hepatic stem cells or hepatic progenitor cells.

A method for introducing the combination as described herein to the cell is not limited. For example, a recombinant vector(s) may be constructed by incorporating nucleic acids when the reprogramming factors are the nucleic acids, or nucleic acids encoding proteins when the reprogramming factors are the proteins into the same or different vector(s), which may be introduced to the cell. The vector(s) may be an expression vector(s) in which the reprogramming factors and/or an additional DNA sequence are operably linked to one or more expression control sequences. Examples of the expression control sequence include promoters, enhancers, and terminators.

The genes encoding the reprogramming factors may be contained as the whole transgene set in one vector or may be contained one by one in separate vectors. Alternatively, some of the genes may be contained in one vector, and the remaining genes may be contained in another vector.

Examples of the expression vector that may be used herein include plasmids and bacteriophages as well as virus vectors derived from retrovirus, vaccinia virus, adenovirus, lentivirus, adeno-associated virus, and hemagglutinating virus of Japan. A method for introducing the vector to the non-hepatic stem cell and the non-hepatic progenitor cell is not particularly limited. Examples thereof include lipofection techniques, electroporation techniques, and calcium phosphate techniques. When the vector is a virus vector, a method of infecting the cell with the virus vector may also be used. The dose of the vector may be appropriately adjusted.

When the reprogramming factors are proteins, the reprogramming factors may be introduced directly to the cell. The reprogramming factors may be produced in a genetic engineering manner (see, for example, "Molecular Cloning, A Laboratory Manual (4th edition)" (Cold Spring Harbor Laboratory Press (2012)). For example, a method of linking the obtained polypeptides to membrane-permeable peptides and introducing the resultants to the cell, or a method of introducing the obtained polypeptides into the cell using cationic lipids may be adopted to the introduction of the polypeptide into the cell, and intracellular delivery reagents are also commercially available (PULSin (Sartorius Polyplus S.A.S. (PPU)), Prote-IN (Hygieia Bioscience (HYG)), BioPORTER Protein Delivery Reagent (Genlantis Inc. (GTS)), etc.).

When the reprogramming factors are low-molecular compounds and penetrate into the cell by contact with the cell, the reprogramming factors may be introduced to the cell by mere contact of the reprogramming factors with the cell. If introduction efficiency into the cell is low, treatment for enhancing the introduction efficiency of the reprogramming factors into the cell may be performed. For example, the low-molecular compounds may be linked to membrane-permeable peptides.

When the reprogramming factors are genes, the genes may be, for example, DNA or RNA (e.g., mRNA). The combination as described herein may be introduced by a method of directly introducing nucleic acids such as mRNA, or a method of introducing them to the cell using lipid nanoparticles (LNP) or the like. In one embodiment, the combination as described herein is contained in lipid nanoparticles (LNP). In one embodiment, the present invention relates to LNP comprising the combination as described herein.

A technique for introducing the reprogramming factors may employ one or more of the combinations described above.

The method for inducing a hepatic stem cell and a hepatic progenitor cell from a non-hepatic stem cell and a non-hepatic progenitor cell, and the method for producing a hepatic stem cell and hepatic progenitor cell from a non-hepatic stem cell and a non-hepatic progenitor cell, as described herein, may be performed *in vitro* or may be performed *in vivo*. In the case of performing the method *in vitro*, the non-hepatic stem cell and the non-hepatic progenitor cell may be cultured for a predetermined period after introduction of the combination as described herein. The culture is performed using a medium for use in animal cell culture, and the cells may be repetitively passaged, if necessary, and thereby reprogrammed into a hepatic stem cell or a hepatic progenitor cell.

In the case of performing the method as described herein *in vivo*, the method may be performed, for example, by introducing the combination as described herein to the non-hepatic stem cell or the non-hepatic progenitor cell from the skin tissue, the blood vessel, or the peritoneal cavity of an animal. In the case of using an expression vector in the introduction of the reprogramming factors, the expression of the reprogramming factors may be rendered tissue-specific by introducing an organ-specific promoter, for example, a liver-specific promoter, to the vector. After introduction of the reprogramming factors, the presence or absence of the cell reprogrammed into a hepatic stem cell or a hepatic progenitor cell may be confirmed, if necessary, in an animal tissue or in cells recovered from the animal tissue. Whether or not the reprogrammed cell thus obtained is a hepatic stem or hepatic progenitor cell may be confirmed on the basis of whether or not the expression of markers expressed in hepatic stem cells or hepatic progenitor cells is increased. Examples of the markers include albumin, α-fetoprotein, and E-cadherin.

In one embodiment, the present invention relates to a method for producing a hepatocyte or a cholangiocyte, comprising : (a) inducing a hepatic stem cell or a hepatic progenitor cell by the method as described herein; and (b) allowing the induced hepatic stem cell or hepatic progenitor cell to differentiate into the hepatocyte or the cholangiocyte.

The differentiation of the hepatic stem cell or the hepatic progenitor cell into the hepatocyte or the cholangiocyte may be performed by a routine method. For example, the hepatic stem cell or the hepatic progenitor cell may be induced to differentiate into the hepatocyte by forming an aggregated mass (aggregate) through three-dimensional suspension culture, and the hepatic stem cell or the hepatic progenitor cell may be induced to differentiate into the cholangiocyte by forming a bile duct-like organoid through embedding in Matrigel and three-dimensional culture. Culture conditions (temperature, period, medium, etc.) may be those used in routine methods, for example according to a method described in WO20198/082874.

In one embodiment, the present invention relates to the combination as described herein or a kit or a composition comprising the combination, for use in the method for inducing a hepatic stem cell or a hepatic progenitor cell from a non-hepatic stem cell or a non-hepatic progenitor cell or the method for producing a hepatic stem cell or a hepatic progenitor cell, as described herein. The kit may comprise one or more of, for example, an instruction, a reagent, and a buffer solution. In the present embodiment, the HNF1A gene and the FOXA3 gene that may be contained in the combination may be contained in a vector(s).

In one embodiment, the present invention relates to a pharmaceutical composition comprising cells, for example, hepatic stem cells or hepatic progenitor cells, obtained by the method for inducing a hepatic stem cell or a hepatic progenitor cell, the method for producing a hepatic stem cell or a hepatic progenitor cell, or the method for producing a hepatocyte or a cholangiocyte, as described herein. In one embodiment, the present invention relates to a method for producing a pharmaceutical composition, comprising: obtaining cells, for example, hepatic stem cells or hepatic progenitor cells, by the method for inducing a hepatic stem cell or a hepatic progenitor cell, the method for producing a hepatic stem cell or a hepatic progenitor cell, or the method for producing a hepatocyte or a cholangiocyte, as described herein; and producing a pharmaceutical composition comprising the obtained cells. The pharmaceutical composition as described herein may be used for the treatment and/or prevention of a disease, for example, liver disease or liver damage. The dosage form, dose, administration frequency, the number of doses, and administration route, for example, of the pharmaceutical composition may be selected depending on the age, sex, body weight, symptoms, and administration route, etc. of a subject.

The pharmaceutical composition as described herein may further contain, in addition to the combination as described herein, a pharmaceutically acceptable carrier (excipient, thickener, binder, lubricant, etc.) and/or a known additive (buffer, tonicity agent, chelating agent, colorant, preservative, fragrance, flavor, sweetener, etc.). For example, lipid nanoparticles (LNP) may be contained as the carrier to thereby promote gene delivery.

### Examples

### <Example 1 Highly efficient preparation of human induced hepatic progenitor cell using Wnt signal activator>

A retrovirus vector (gene expression vector) containing human iHepPC inducing factors (FOXA3, HNF1A, and HNF6) was introduced to human umbilical vein endothelial cells (HUVEC) (Takara Bio Inc.) by retrovirus infection. The culture of the cells, the preparation of the retrovirus, and the retrovirus infection of the cells were performed as described in Hiroki Inada et al., Nature Communications, Vol. 11, Article number: 5292, 2020. Six hours after retrovirus infection, the Wnt signal activator CHIR99021 (Tocris Bioscience): 3 µM or BIO (6-bromoindirubin-3-oxime) (Calbiochem): 1 µM was added to the culture solution, and albumin (ALB) immunostaining and colony number counting of formed colonies were performed on days 9 and 16. A group supplemented with no Wnt signal activator was expressed as Control. DNA of the cells was stained with DAPI (blue). The immunostaining was performed in accordance with Hiroki Inada et al. (supra). The colony counting was performed by counting colonies observed under a microscope or in a microscopically taken photograph.

The results are shown in Figure 1. Figure 1A shows results of the immunostaining, and Figure 1B shows results of the colony number counting. Figure 1 shows that the human iHepPC inducing factors (FOXA3, HNF1A, and HNF6) plus the Wnt signal activator CHIR99021 or BIO increased the number of cells expressing the hepatic progenitor cell marker albumin and reduced the time for the albumin-expressing cells to appear, i.e., enhanced induction efficiency into human hepatic progenitor cells.

### <Example 2 Decrease in human iHepPC colony formation frequency by addition of Wnt signal inhibitor to culture solution>

A retrovirus vector (gene expression vector) containing human iHepPC inducing factors (FOXA3, HNF1A, and HNF6) was introduced to HUVEC by retrovirus infection. The culture of the cells, the preparation of the retrovirus, and the retrovirus infection of the cells were as described in Example 1. Six hours after retrovirus infection, the Wnt signal inhibitor (IWP2 (Nacalai Tesque, Inc.): 0.5 µg/ml) was added to the culture solution, and albumin (ALB) immunostaining (A) and ALB gene expression analysis (B) of formed colonies were performed on days 9, 16, 23, and 44. A group supplemented with no Wnt signal inhibitor was expressed as Control. DNA of the cells was stained with DAPI (blue). The immunostaining method was performed in accordance with Example 1, and the gene expression analysis was performed in accordance with Hiroki Inada et al. (supra).

The results are shown in Figure 2. Figure 2 shows that the human iHepPC inducing factors (FOXA3, HNF1A, and HNF6) plus the Wnt signal inhibitor IWP2 decreased the number of cells expressing the hepatic progenitor cell marker albumin, i.e., decreased induction efficiency into human hepatic progenitor cells.

### <Example 3 Substitution of HNF6 by Wnt signal activator>

The presence or absence of human iHepPC induction was tested in three groups: a group 1) in which human iHepPC inducing factors (FOXA3, HNF1A, and HNF6) were introduced to HUVEC, a group 2) in which some of the human iHepPC inducing factors (FOXA3 and HNF1A) were introduced thereto, and a group 3) in which some of the human iHepPC inducing factors (FOXA3 and HNF1A) were introduced thereto, followed by the addition of the Wnt signal activator (CHIR99021: 3 µM) to a culture solution 6 hours later. After introduction of the iHepPC inducing factors, cell culture for 1 month and subsequent immunostaining were performed. The immunostaining was performed as described in Example 1.

The results are shown in Figure 3. Human iHepPC positive to albumin (ALB) and E-cadherin (E-CAD) in immunostaining was induced only in the group 1) and the group 3), whereas no ALB- and E-CAD-positive human iHepPC was induced in the group 2). This indicates that FOXA3 and HNF1A alone are insufficient for the induction of human iHepPC, which further requires HNF6; and HNF6 may be substituted by the Wnt signal activator.

## Claims

1. A method for inducing a hepatic stem cell or a hepatic progenitor cell from a non-hepatic stem cell or a non-hepatic progenitor cell, comprising
introducing a combination comprising:
HNF1 gene or HNF1 protein, FOXA gene or FOXA protein, and a Wnt signal activator to the non-hepatic stem cell or the non-hepatic progenitor cell.

2. A method for producing a hepatic stem cell or a hepatic progenitor cell, comprising
inducing the hepatic stem cell or the hepatic progenitor cell from a non-hepatic stem cell or a non-hepatic progenitor cell by introducing a combination comprising:
HNF1 gene or HNF1 protein, FOXA gene or FOXA protein, and a Wnt signal activator to the non-hepatic stem cell or the non-hepatic progenitor cell.

3. The method according to claim 1, wherein the combination consists of the HNF1 gene or the HNF1 protein, the FOXA gene or the FOXA protein, and the Wnt signal activator.

4. The method according to claim 1, wherein HNF1 is HNF1A.

5. The method according to claim 1, wherein FOXA is FOXA3.

6. The method according to claim 1, wherein the Wnt signal activator is CHIR99021 or BIO (6-bromoindirubin-3-oxime).

7. The method according to claim 6, wherein the Wnt signal activator is CHIR99021.

8. The method according to claim 1, wherein the combination further comprises HNF6 gene or HNF6 protein.

9. A method for producing a hepatocyte or a cholangiocyte, comprising:
(a) inducing a hepatic stem cell or a hepatic progenitor cell by a method according to claim 1; and
(b) allowing the induced hepatic stem cell or hepatic progenitor cell to differentiate into the hepatocyte or the cholangiocyte.

10. A combination for use in a method according to claim 1 or 2, comprising:
HNF1 gene or HNF1 protein, FOXA gene or FOXA protein, and a Wnt signal activator.
